(19) Europäisches Patentamt
European Patent Office
Office européen des brevets

(11) Veröffentlichungsnummer: **0 352 672 A2**

(12) # EUROPÄISCHE PATENTANMELDUNG

(21) Anmeldenummer: **89113499.1**

(51) Int. Cl.[4]: **C07C 47/548**

(22) Anmeldetag: **22.07.89**

(30) Priorität: **28.07.88 DE 3825577**
**28.07.88 DE 3825578**

(43) Veröffentlichungstag der Anmeldung:
**31.01.90 Patentblatt 90/05**

(84) Benannte Vertragsstaaten:
**BE CH DE ES FR GB LI**

(71) Anmelder: **BASF Aktiengesellschaft**
**Carl-Bosch-Strasse 38**
**D-6700 Ludwigshafen(DE)**

(72) Erfinder: **Hickmann, Eckhard, Dr.**
**Kantstrasse 23**
**D-6701 Dannstadt-Schauernheim(DE)**
Erfinder: **Hoelderich, Wolfgang, Dr.**
**Mannheimer Strasse 18 c**
**D-6701 Frankenthal(DE)**

(54) **Verfahren zur Herstellung von Alkenylaromaten.**

(57) Die Anmeldung betrifft ein Verfahren zur Herstellung von Alkenylaromaten der allgemeinen Formel I

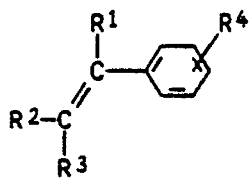

(I),

in der $R^1$ geradkettige oder verzweigte Alkylreste mit 1 bis 12 Kohlenstoffatomen oder Cycloalkylreste mit 5 bis 8 Kohlenstoffatomen oder Arylreste bedeuten kann, in der $R^2$ und $R^3$ gleich oder verschieden sind und Wasserstoff oder Reste wie unter $R^1$ genannt sein können, und in der $R^4$ für Wasserstoff, Kohlenwasserstoffreste wie $R^1$, Alkenylreste mit 2 bis 12 Kohlenstoffatomen, Alkoxireste mit 1 bis 12 Kohlenstoffatomen, Aryloxireste mit 6 bis 12 Kohlenstoffatomen, der Formylrest, Acylreste mit 2 bis 12 Kohlenstoffatomen, Acetalreste mit 3 bis 12 Kohlenstoffatomen, Esterreste mit 2 bis 12 Kohlenstoffatomen, der Cyanorest, Halogen oder einen Halogenalkylrest mit 1 bis 5 Kohlenstoffatomen, z.B. $-CF_3$, $-CCl_3$, $-CH_2CF_3$, steht, indem man Alkoxialkylaromaten der allgemeinen Formel II

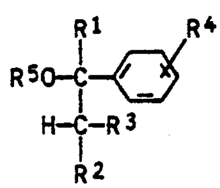

(II),

in der $R^1$ bis $R^4$ obige Bedeutung haben und $R^5$ für geradkettige oder verzweigte Alkylreste steht, in Gegenwart von aciden Heterogenkatalysatoren umsetzt.

EP 0 352 672 A2

## Verfahren zur Herstellung von Alkenylaromaten

Die Erfindung betrifft ein Verfahren zur Herstellung von Alkenylaromaten durch Abspaltung von Alkoholen aus Alkoxialkylaromaten in Gegenwart von Zeolithen und/oder Phosphaten mit Zeolithstruktur als Katalysatoren.

Die erfindungsgemäßen Substanzen sind wertvolle Zwischenprodukte für Wirkstoffe und Polymere. Derartige Verbindungen können bislang oft nur auf sehr umständliche und unwirtschaftliche Weise hergestellt und isoliert werden:

Zum Beispiel wurde p-Isopropenylbenzaldehyd bisher auf folgenden Wegen hergestellt:

a) Oxidation von Limonen mit Selendioxid (Chem. Abstr. Vol. 57, 9884 d);

b) Umsetzung der Grignard-Verbindung aus p-Isopropenylchlorbenzol mit Orthoameisensäuretrimethylester und Verseifung des erhaltenen p-Isopropenylbenzaldehyddimethylacetals (Chem. Abstr. Vol. 65, 15 521 f);

c) Umsetzung der gleichen Grignard-Verbindung mit Dimethylformamid (Chem. Abstr. Vol. 65; 15 299 f);

d) Tris-(triphenylphosphin)-rutheniumdichlorid-katalysierte Abspaltung von 2-Methyl-but-2-en aus dem p-Isopropenylbenzyl-2-methyl-but-3-en-2-yl-ether (J. Amer. Chem. Soc. 1977, 99, 4372);

e) Umsetzung von p-Bromphenyl-magnesiumbromid mit Aceton, Wasserabspaltung zum p-Isopropenylbrombenzol, Grignardierung und Umsetzung mit Dimethylformamid (Helv. Chim. Acta, 1981, 64, 2393).

All diese Synthesen kommen für eine Herstellung unter technischen Bedingungen nicht in Frage.

Es ist auch bekannt, daß man Vinylether durch Abspaltung von Alkoholen aus Acetalen an Aluminosilikatzeolithen erhalten kann. Diese Zeolithe zeigen in ihrer aciden H-Form Selektivitäten und Umsätze, die zu wünschen übrig lassen. Durch einen zusätzlichen Verfahrensschritt einer Na-Dotierung des Zeolithen ist es möglich, die Selektivität bei nicht vollständigem Umsatz zu steigern.

Die bekannten Verfahren haben den Nachteil, daß sie z.B. Folgereaktionen mit der Doppelbindung, den Aromaten oder den Substituenten eingehen können.

Es bestand daher die Aufgabe, Alkenylaromaten ohne Folgereaktionen an der Doppelbindung, an Aromaten oder an den Substituenten auf einfachem Wege aus leicht zugänglichen Ausgangsstoffen herzustellen, wobei möglichst vollständiger Umsatz, hohe Selektivität und lange Standzeit des Katalysators angestrebt wurden.

Demgemäß wurde ein Verfahren zur Herstellung von Alkenylaromaten der allgemeinen Formel I

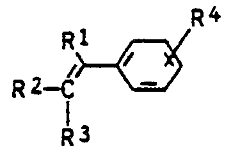

(I),

in der $R^1$ geradkettigte oder verzweigte Alkylreste mit 1 bis 12 Kohlenstoffatomen oder Cycloalkylreste mit 5 bis 8 Kohlenstoffatomen oder Arylreste bedeutet, $R^2$ und $R^3$ gleich oder verschieden sind und Wasserstoff oder Reste wie unter $R^1$ genannt sein können, und in der $R^4$ für Wasserstoff, Kohlenwasserstoffreste wie $R^1$, Alkenylreste mit 2 bis 12 Kohlenstoffatomen, Alkoxireste mit 1 bis 12 Kohlenstoffatomen, Aryloxireste mit 6 bis 12 Kohlenstoffatomen, der Formylrest, Acylreste mit 2 bis 12 Kohlenstoffatomen, Acetalreste mit 3 bis 12 Kohlenstoffatomen, Esterreste mit 2 bis 12 Kohlenstoffatomen, der Cyanorest, Halogen oder einen Halogenalkylrest mit 1 bis 5 Kohlenstoffatomen, z.B. $-CF_3$, $-CCl_3$, $-CH_2CF_3$, steht, gefunden, welches dadurch gekennzeichnet ist, daß man Alkoxialkylaromaten der allgemeinen Formel II,

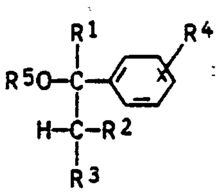

(II),

in der $R^1$ bis $R^4$ obige Bedeutung haben und $R^5$ für geradkettige oder verzweigte Alkylreste steht, in

Gegenwart von aciden Heterogenkatalysatoren umsetzt.

Bei dem erfindungsgemäßen Verfahren werden die an die Katalysatoren gestellten Anforderungen weitgehend erfüllt. Nach dem Stand der Technik war dies überraschend, weil die bisherige Lehre gerade in entgegengesetzter Richtung, nämlich die Ausschaltung von aciden Zentren durch die Natriumdotierung, wies. Daher konnte man nicht erwarten, daß man mit den erfindungsgemäß eingesetzten sauren Katalysatoren in so weiten Grenzen so hervorragende Ergebnisse erhalten würde. Auch hätte man bei den hohen Temperaturen in der Gasphase mit Folgereaktionen wie Oligomerisierung oder Crackreaktionen rechnen müssen. Bei dem neuen Verfahren werden keine schwer zugänglichen Ausgangsstoffe, keine giftigen Katalysatoren und keine mehrstufige Verfahrensführung angewendet.

Als Rest $R^1$ kommen unabhängig von den anderen Resten Kohlenwasserstoffreste n-$C_nH_{2n+1}$ (n = 1-12), verzweigte Kohlenwasserstoffreste i-$C_nH_{2n+1}$ (n = 1-12), Cycloalkylreste mit 5-8 Kohlenstoffatomen oder Arylreste in Betracht.

Die Reste $R^2$ und $R^3$ können gleich oder verschieden sein und haben die Bedeutung von Wasserstoff oder von Kohlenwasserstoffen wie bei $R^1$ genannt.

$R^4$ steht für Wasserstoff, Kohlenwasserstoffreste wie $R^1$, Alkenylreste mit 2 bis 12 Kohlenstoffatomen, Alkoxireste mit 1 bis 12 Kohlenstoffatomen, Aryloxireste mit 6 bis 12 Kohlenstoffatomen, der Formylrest, Acylreste mit 2 bis 12 Kohlenstoffatomen, Acetalreste mit 3 bis 12 Kohlenstoffatomen, Esterreste mit 2 bis 12 Kohlenstoffatomen, der Cyanorest, Halogen oder einen Halogenalkylrest mit 1 bis 5 Kohlenstoffatomen, z.B. $-CF_3$, $-CCH_3$, $-CH_2CF_3$, steht.

Als Rest $OR^5$ kommen z.B. Methoxi-, Ethoxi-, n-/i-Propoxi-, n-/i-/t-Butoxi-Hexoxireste in Betracht.

Beispiele für mögliche Edukte sind etwa:

Alkoxiisopropylbenzaldehyde, insbesondere p-Alkoxiisopropylbenzaldehyde mit Alkoxi = Methoxi, Ethoxi, Propoxi-, i-Propoxi, n- und verzweigt-Butoxi, Alkoxiisopropylbenzaldehyd-dialkylacetale, insbesondere p-Alkoxiisopropylbenzaldehyd-dialkylacetale, vorzugsweise solche, in denen alle Alkoxigruppen gleich sind und identisch mit den oben aufgeführten Alkoxigruppen, Alkoxiisopropyltoluole, insbesondere p-Alkoxiisopropyltoluole mit den o.a. Alkoxigruppen, im Aromaten Halogen-, Nitril- und Ester-substituierte Alkoxiisopropylaromaten, z.B. p-Methoxiisopropyl-brombenzol, p-Methoxiisopropylbenzonitril und p-Methoxiisopropylbenzoesäuremethylester, 1-Alkoxi-1-arylcyclohexane und 1-Alkoxi-1-arylcyclopentane, z.B. p-Methoxi-1-phenylcyclohexan oder 1-Ethoxi-1-p-tolylcyclopentan.

Wegen der im allgemeinen höheren Regioselektivität der Eliminierungsreaktion ist es vorteilhaft, wenn der Alkoxialkylrest symmetrisch ist; die Reaktion ist aber auch auf Verbindungen mit unsymmetrischen Alkoxialkylresten anwendbar.

Als Katalysatoren für das erfindungsgemäße Verfahren verwendet man acide Heterogenkatalysatoren, insbesondere saure Oxide, Phosphate, die gegebenenfalls Zeolithstruktur besitzen und/oder Zeolithe.

Phosphate sind z.B. Aluminiumphosphate, Siliciumaluminiumphosphate, Cerphosphate, Zirkonphosphate, Borphosphate, Eisenphosphate, Strontiumhydrogenphosphat oder deren Gemische.

Als Phosphatkatalysatoren kann man bei dem Verfahren gefällte Phosphate, z.B. gefällte Aluminiumphosphate einsetzen. Ein derartiges Aluminiumphosphat wird beispielsweise hergestellt, indem 92 g Diammoniumhydrogenphosphat in 700 ml gelöst werden . Zu dieser Lösung wird 260 g $Al(NO_3)_3 \times H_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60°C/16 h getrocknet.

Geeignete Borphosphate kann man beispielsweise durch Mischen und Kneten von konzentrierter Borsäure und Phosphorsäure und durch anschließende Trocknung und Calcination in Inertgas-, Luft- oder Dampfatmosphäre bei 250 bis 650°C, vorzugsweise 300 bis 500°C, herstellen.

Auf diese Phosphate können auch durch Imprägnierung (Tränken und Aufsprühen) Modifizierungskomponenten aufgebracht werden. Auch kann eine Modifizierung mit Säuren erfolgen.

Die so hergestellten Phosphate können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110°C/16 h getrocknet und bei 500°C/16 h calciniert.

Man erhält auch vorteilhafte Katalysatoren, wenn das isolierte Phosphat direkt nach der Trocknung verformt wird und erst nach der Verformung einer Calcinierung unterworfen wird. Die hergestellten Phosphate können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure. Ammoniak, Amine, Silikoester und Graphit oder deren Gemische verwendet werden.

Um eine möglichst hohe Selektivität, hohen Umsatz sowie lange Standzeiten zu erreichen, ist es vorteilhaft, die Phosphate zu modifizieren. Eine geeignete Modifizierung der Katalysatoren besteht z.B. darin, daß man das unverformte oder verformte Phosphat mit Metallsalzen durch Imprägnierung dotiert. Als Metalle werden Alkalimetalle wie Li, Cs, K, Erdalkalimetalle wie Mg, Ca, Sr, Metalle oder 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Ir, Ni, Pd, Pt, Übergangsmetalle der 1. und 2. Nebengruppe wie Cu, Ag, Zn, Seltene Erdmetalle wie La, Ce, Pr, Nd, Pr, Yb und U eingesetzt.

Eine Möglichkeit der Metallaufbringung ist gegeben, indem man das Phosphat z.B. mit einem Halogenid, einem Nitrat oder einemn Oxid der voranbeschriebenen Metalle in wäßriger, alkoholischer oder ammoniakalischer Lösung imprägniert. An eine Imprägnierung schließt sich zumindest eine Trocknung, wahlweise eine abermalige Calcinierung an.

Eine mögliche Ausführungsform besteht z.B. darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser löst. Mit dieser Lösung wird das verformte oder unverformte Phosphat eine gewisse Zeit, etwa 30 Minuten, getränkt. Die eventuell überstehende Lösung wird am Rotationsverdampfer von Wasser befreit. Danach wird das getränkte Phosphat bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, z.B. eine wäßrige $Ni(CO_3)_2$-Lösung oder ammoniakalische $Pd(NO_3)_2$-Lösung herzustellen und darin das reine pulverförmige Phosphat bei 40 bis 100 °C unter Rühren etwa 24 h aufzuschlämmen. Nach Abfiltrieren, Trocknen bei etwa 150 °C und Calcinierung bei etwa 500 °C kann das so gewonnene Material mit oder ohne Bindemittel zu Strängen, Pellets oder Wirbelgut weiterverarbeitet werden.

Bei manchen metalldotierten Phosphaten, z.B. bei Pd-, Cu-, Ni-dotierten Phosphaten ist eine Nachbehandlung mit Wasserstoff vorteilhaft.

Eine weitere Möglichkeit der Modifizierung besteht darin, daß man das Phosphat - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man das Phosphat in Pulverform mit 1 n Phosphorsäure 1 Stunde bei 80 °C behandelt. Nach der Behandlung wird mit Wasser gewaschen, bei 110 °C/16 h getrocknet und bei 500 °C/20 h calciniert. Nach einer anderen Arbeitsweise behandelt man das Phosphat vor oder nach seiner Verformung mit Bindemitteln, z.B. 1 bis 3 Stunden bei Temperaturen von 60 bis 80 °C mit einer 3 bis 25 Gew.-%igen wäßrigen Salzsäure. Anschließend wird das so behandelte Phosphat mit Wasser gewaschen, getrocknet und bei 400 °C bis 500 °C calciniert. Einer HF-Behandlung kann sich auch eine HCl-Behandlung anschließen.

Geeignete saure Katalysatoren sind z.B. auch die sauer wirkenden Oxide von Elementen der III. und IV. Hauptgruppe sowie der IV. bis VI. Nebengruppe des Periodischen Systems, insbesondere Oxide wie Siliciumdioxid in Form von Kieselgel, Kieselgur, weiterhin Titandioxid, Zirkondioxid, Phosphoroxide, Vanadiumoxide, Nioboxide, Boroxide, Aluminiumoxide, Chromoxide, Molybdänoxide, Wolframoxide oder Bims oder Gemische dieser Oxide. Auch können diese Oxide durch Aufbringung von Modifizierungskomponenten, wie vorangehend beschrieben, dotiert werden. Die Behandlung mit Säuren, wie oben beschrieben, ist ebenfalls eine Möglichkeit der Modifizierung.

Auch mit Phosphorsäure oder Borsäure getränkte Katalysatoren können eingesetzt werden. Phosphorsäure oder Borsäure wird z.B. auf $SiO_2$-, $Al_2O_3$- oder Bimsträger z.B. durch Auftränken oder Versprühen aufgebracht. Ein phosphorsäurehaltiger Katalysator kann beispielsweise durch Auftränken von $H_3PO_4$- oder $NaH_2PO_4$- oder $Na_2HPO_4$-Lösung auf $SiO_2$ und anschließende Trocknung bzw. Calcination erhalten werden. Phosphorsäuren kann aber auch mit Kieselgel zusammen in einem Sprühturm versprüht werden; danach schließt sich eine Trocknung und meist eine Calcination an. Phosphorsäure kann auch in einer Imprägniermühle auf das Trägermaterial aufgesprüht werden.

Ferner werden als Katalysatoren für das erfindungsgemäße Verfahren acide zeolithische Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein

vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedliche große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Hierzu gehören auch die iso taktischen Zeolithe nach EP-A-34 727 und EP-A-46 504. Die erhaltenen Aluminosilikatzeolithe enthalten je nach Wahl der Ausgangsstoffmengen ein $SiO_2/Al_2O_3$-Verhältnis von 10 bis 40.000. Diese Aluminosilikatzeolithe kann man auch in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter autogenem Druck synthetisiert, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP-A-34 727 und EP-A-46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Zu den verwendbaren siliciumreichen Zeolithen ($SiO_2/Al_2O_3 \geq 10$) gehören auch die sog. ZSM-Typen, Ferrierit, NU-1 und Silicalit®, ein Molekularsieb, ein sog. Silica Polymorph.

Die so hergestellten Alumino, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnbis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Um eine möglichst hohe Selektivität, hohen Umsatz sowie lange Standzeiten zu erreichen, is es vorteilhaft, die Zeolithe zu modifizieren. Eine geeignete Modifizierung der katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. bis 8. Nebengruppe wie Ti, Zr, V, Nb, Cr, Mo, W, Mn, Re, Fe, Ru, Os, Co, Rh, Ir, Ni, Pd, Pt, Übergangsmetalle der 1. und 2. Nebengruppe wie Cu, Ag, Zn, Seltene Erdmetalle wie La, Ce, Pm, Nd, Pr, Yb und U eingesetzt.

Zweckmäßigerweise führt man die Dotierung so durch, daß man z.B. den verformten Zeolithen in einem Steigrohr vorlegt und bei 20 bis 100 °C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- oder Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht, z.B. darin, daß man $Cu(NO_3)_2 \times 3\ H_2O$ oder $Ni(NO_3)_2 \times 6\ H_2O$ oder $La(NO_3)_2 \times 6\ H_2O$ oder $Cs_2CO_3$ in Wasser löst. Mit dieser Lösung wird der verformte oder unverformte Zeolith eine gewisse Zeit, etwa 30 Minuten, getränkt. Die eventuell überstehende Lösung wird am Rotationsverdampfer von Wasser befreit. Danach wird der getränkte Zeolith bei etwa 150 °C getrocknet und bei etwa 550 °C calciniert. Dieser Tränk vorgang kann mehrmals hintereinander vorgenommen werden, um den gewünschten Metallgehalt einzustellen.

Es ist auch möglich, z.B. eine wäßrige $Ni(CO_3)_2$-Lösung oder ammoniakalische $Pd(NO_3)_2$-Lösung herzustellen und darin den reinen pulverförmigen Zeolithen bei 40 bis 100 °C unter Rühren etwa 24 h aufzuschlämmen. Nach Abfiltrieren, Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80 °C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolithische Material vor seiner Verformung bei erhöhter Temperatur mit Flußsäure, die im allgemeinen als 0,01 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160 °C, getrocknet und bei Tempe raturen von im allgemeoinen 450 bis 600 °C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90 °C, vorzugsweise 60 bis 80 °C, über einen Zeitraum von 0,5 bis 5 h, vorzugsweise mit 12 bis 20 gew.%iger Salzsäure, behandelt. Anschließend wird das zeolithische Material im allgemeinen ausgewaschen und zweckmäßig, z.B. bei Temperaturen von 100 bis 160 °C, getrocknet und bei Temperaturen von im allgemeinen 450 bis 600 °C calciniert. Einer HF-Behandlung kann sich auch eine HCl-Behandlung anschließen.

Nach einer anderen Arbeitsweise lassen sich Zeolithe durch Aufbringung von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primären Natriumphosphat erwiesen. Hierbei

werden die Zeolithe in Strang-, Tabletten- oder Wirbelgut-Form mit wäßriger $NaH_2PO_4$-Lösung getränkt, bei 100 °C getrocknet und bei 500 °C calciniert.

Weitere Katalysatoren für das erfindungsgemäße Verfahren sind Phosphate mit Zeolithstruktur, insbesondere Aluminiumphosphate, Siliciumaluminiumphosphate, Siliciumeisenaluminiumphosphate, Zirkonphosphate, Siliciumborphosphat oder deren Gemische.

Als Aluminiumphosphat-Katalysatoren werden für das erfindungsgemäße Verfahren unter hydrothermalen Bedingungen synthetisierte Aluminiumphosphate eingesetzt.

Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP 132 708, US 4 310 440 und US 4 473 663 beschrieben.

Beispielsweise das $AlPO_4$-5 (APO-5) wird synthetisiert, indem man Orthophosphorsäure mit Pseudoboehmit (Catapal SB®) in Wasser homogen mischt, zu dieser Mischung Tetrapropylammoniumhydroxid gibt und danach bei ca. 150 °C 20 bis 60 h unter autogenem Druck in einem Autoklaven umsetzt. Das abfiltrierte $AlPO_4$ wird getrocknet bei 100 bis 160 °C und calciniert bei 450 bis 550 °C.

$AlPO_4$-9 (APO-9) wird ebenfalls aus Orthophosphorsäure und Pseudoboehmit, aber in wäßriger DABCO-Lösung (1,4-Diazobicyclo-(2,2,2)octan) bei ca. 200 °C unter autogenem Druck während 200 bis 400 h synthetisiert.

Die Synthese des $AlPO_4$-21 (APO-21) erfolgt aus Orthophosphorsäure und Pseudoboehmit in wäßriger Pyrrolidon-Lösung bei 150 bis 200 °C unter autogenem Druck während 50 bis 200 h.

Die für das erfindungsgemäße Verfahren eingesetzten Siliciumaluminiumphosphate sind z.B. SAPO 5, SAPO-11, SAPO-31 und SAPO-34. Die Synthese dieser Verbindung wird z.B. in EP 103 117, US 4 440 871 beschrieben. SAPO's werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$, suspendiert in wäßriger Tetrapropylammoniumhydroxid-Lösung, mit einer wäßrigen Suspension aus Pseudoboehmit und Orthophosphorsäure und anschließender Umsetzung bei 150 bis 200 °C während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C.

Auf diese Phosphate können durch Imprägnierung (Tränken und Aufsprühen) oder in manchen Fällen auch durch Ionenaustausch Modifizierungskomponenten, wie voran bei den Zeolithen beschrieben, aufgebracht werden. Auch kann wie bei den Zeolithkatalysatoren eine Modifizierung mit Säuren erfolgen.

Wenn bei der Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die Reaktion in der bevorzugten Gasphase wird zweckmäßig bei 100 bis 500 °C, vorzugsweise 200 bis 400 °C, und einer Belastung WHSV = 0,1 bis 20 $h^{-1}$, bevorzugt 0,5 bis 5 $h^{-1}$ (g Einsatzgemisch/g Katalysator und Stunde) durchgeführt. Die Reaktion kann im Festbett oder im Wirbelbett ausgeführt werden.

Es ist auch möglich, die Reaktion in der Flüssigphase (Suspensions-, Riesel- oder Sumpffahrweise) bei Temperaturen zwischen 50 und 200 °C durchzuführen.

Das Verfahren kann bei Normaldruck, bei vermindertem oder erhöhtem Druck diskontinuierlich, vorzugsweise kontinuierlich durchgeführt werden. Insbesondere die Verfahrensweise bei vermindertem Druck kann vorteilhaft sein.

Schwerflüchtige oder feste Edukte werden in gelöster Form, z.B. in THF-, Toluol- oder Petrolether-Lösung zum Einsatz gebracht. Generell ist eine Verdünnung des Eduktes mit derartigen Lösungsmitteln oder mit Inertgasen wie $N_2$, Ar, $H_2O$-Dampf möglich.

Die Ausführung der Reaktion mit Wasser oder Wasserdampf ermöglicht den direkten Zugang zum aromatischen Aldehyd aus dem entsprechenden Acetal.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Vorzugsweise werden die gasförmigen Reaktionsprodukte sofort in eine Trennung eingebracht und

anschließende in ihre Einzelkomponenten zerlegt. Eine derartige Trennung kann z.B. in einer Fraktionierkolonne durchgeführt werden. Dies kann eine Rückreaktion unterbinden und einen hohen Umsatz bewirken.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit einem Verformungshilfsmittel 2-mm-Stränge hergestellt, die bei 110 °C/16 h getrocknet und bei 500 °C/24 h calciniert werden.

Katalysator B

Ein Aluminosilikatzeolith vom Pentasil-Typ wird unter hydrothermalen Bedingungen bei autogenem Druck und 150 °C aus 65 g hochdispersam $SiO_2$, 20,3 g $Al_2(SO_4)_3$ x 18 $H_2O$ in 1 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Aluminosilikatzeolith enthält 91,6 Gew.% $SiO_2$ und 4,6 Gew.% $Al_2O_3$. Der Katalysator wird mit einem Verformungshilfsmittel zu 2-mm-Strängen verformt, bei 110 °C/16 h getrocknet und bei 500 °C/24 h calciniert.

Katalysator C

Katalysator C wird erhalten, indem man die Stränge des Katalysators A mit einer wäßrigen $Cs_2O_3$-Lösung imprägniert, danach bei 130 °C/2 h trocknet und bei 540 °C/2 h calciniert. Der Cs-Gehalt beträgt 0,6 Gew.%.

Katalysator D

200 g des bei Katalysator A beschriebenen Borosilikatzeolithes werden mit 1 l einer wäßrigen Lösung aus 16,7 g $FeCl_3$ x 6 $H_2O$ und 50 g $NH_4Cl$ 24 h bei Raumtemperatur ionenausgetauscht, danach gründlich mit $H_2O$ Cl-frei gewaschen, bei 150 °C/1 h getrocknet und 500 °C/2 h calciniert. Dieses Pulver wird mit hochdispersem $SiO_2$ im Gewichtsverhältnis 70:30 verformt. Nach Trocknung werden die Stränge bei 500 °C/16 h calciniert.

Katalysator E

Katalysator E wird erhalten, indem man die Stränge des Katalysators A mit einer wäßrigen Lösung aus Cer- und Palladiumnitrat imprägniert, danach bei 130 °C/2 h trocknet und bei 540 °C/2 h calciniert. Der Ce-Gehalt beträgt 2,3 Gew.%, der Pd-Gehalt 0,5 Gew.%.

Katalysator F

Katalysator F wird wie Katalysator C hergestellt, wobei jedoch $Cs_2CO_3$ durch $Fe(NO_3)_3$ ersetzt wird. Der Fe-Gehalt beträgt 2,9 Gew.%.

Katalysator G

Der Eisensilikatzeolith des Pentasil-Typs wird unter hydrothermalen Bedingungen bei autogenem Druck und 165°C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110°C/24 h getrocknet und bei 500°C/24 h calciniert. Man erhält einen Eisensilikatzeolithen mit einem $SiO_2/Fe_2O_3$-Verhältnis von 17,7 und einen $Na_2O$-Gehalt von 1,2 Gew.%. Der Katalysator wird mit hochdispersem $SiO_2$ im Gewichtsverhältnis 70:30 zu 2,5-mm-Strängen verstrangt, bei 110°C/16 h getrocknet und bei 500°C/24 h calciniert. Diese Stränge werden mit einer 20 %igen $NH_4Cl$-Lösung bei 80°C ionenausgetauscht und danach chloridfrei gewaschen, bei 110°C getrocknet und bei 500°C/5 h calciniert. Der Ionenaustausch wird so lange vorgenommen, bis der Na-Gehalt 0,002 Gew.% beträgt.

Katalysator H

Katalysator H wird wie Katalysator C hergestellt, wobei jedoch $Cs_2CO_3$ durch $Ce(NO_3)_2$ ersetzt wird. Der Ce-Gehalt beträgt 1,7 Gew.%.

Katalysator I

Katalysator I wird wie Katalysator C hergestellt, wobei jedoch $Cs_2CO_3$ durch $Cr(NO_3)_3$ ersetzt wird. Der Cr-Gehalt beträgt 2,2 Gew.%.

Katalysator J

$AlPO_4$-12 (APO-12) wird synthetisiert, indem man 200 g 98 %ige Phosphorsäure und 136 g Boehmit in 400 g Wasser löst bzw. suspendiert, hierzu eine wäßrige Lösung aus 60 g Ethylendiamin und 320 g $H_2O$ zugibt und diese Mischung in einem Rührautoklaven bei 200°C während 24 Stunden unter autogenem Druck umsetzt. Nach Abfiltrieren wird das kristalline Material bei 120°C getrocknet und bei 500°C/16 h calciniert. Das so synthetisierte $AlPO_4$-12 enthält 55,5 Gew.% $P_2O_5$, 39,7 Gew.% $Al_2O_3$. Dieses Material wird mit Verstrangungshilfsmitteln zu 3-mm-Strängen verformt, abermals bei 120°C getrocknet und bei 500°C/6 h calciniert.

Katalysator K

Die Synthese von $AlPO_4$-5 (APO-5) erfolgt durch Zusammenrühren von 200 g 95 %iger Phosphorsäure, gelöst in 325 g $H_2O$, 136 g Boehmit und 678 g Tetrapropylammoniumhydroxid (30 %ig) und anschließende Reaktion bei 150°C unter autogenem Druck während 43 Stunden. Das bei 120°C getrocknete und bei 500°C/16 h calcinierte Produkt enthält 46,5 Gew.% $P_2O_5$ und 45,5 Gew.% $Al_2O_3$. Dieses $AlPO_4$-5 wird mit Boehmit im Gewichtsverhältnis 60:40 zu 2-mm-Strängen verformt, bei 110°C getrocknet und bei 500°C/16 h calciniert.

Katalysator L

Siliciumaluminiumphosphat-5 (SAPO-5) wird hergestellt aus einer Mischung aus 200 g 98 %iger Phosphorsäure, 136 g Boehmit, 60 g Kieselsol (30 %ig) 287 g Tripropylamin und 587 g $H_2O$. Diese Mischung wird bei 150°C während 168 Stunden unter autogenem Druck umgesetzt. Nach Filtration wird das kristalline Produkt bei 120°C getrocknet und bei 500°C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3-mm-Strängen verformt, bei 120°C getrocknet und bei 500°C calciniert.

Katalysator M

Kommerziell erhältlicher L-Zeolith (Baylith L®) wird mit Boehmit im Gewichtsverhältnis 80:20 zu 2-mm-Strängen verformt. Nach Trocknung bei 110 °C/16 h und Calcination bei 500 °C/16 h liegt der fertige Katalysator M vor.

Katalysator N

Kommerziell erhältlicher NaY-Zeolith wird mit Verformungshilfsmittel zu 2-mm verstrangt, bei 110 °C getrocknet und bei 500 °C/16 h calciniert und mit 20 %iger Ammoniumchloridlösung einem Ionenaustausch unterworfen. Der Restnatriumgehalt des Katalysators N beträgt 0,07 Gew.% (500 °C calciniert.)

Die mit diesen Katalysatoren erzielten Versuchsergebnisse und Versuchbedingungen sind in den nachfolgenden Beispielen und in Tabelle 1 zusammengefaßt.

Katalysator P

Kommerziell erhältliches Zirkonphosphat $Zr_3(PO_4)_4$ in Reinsubstanz verformt.

Katalysator Q

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120 °C getrocknet und bei 450 °C calciniert. Katalysator Q enthält 47,1 Gew.% Ce und 12,7 Gew.% P.

Katalysator R

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3-mm-Strängen verformt. Diese Stränge werden bei 100 °C getrocknet und bei 350 °C calciniert. Katalysator R enthält 8,77 Gew.% B und 28,3 Gew.% P.

Katalysator S

Katalysator S ist ein gefälltes Aluminiumphosphat, das durch Fällung aus $Al(NO_3)_3$-$H_3PO_4$-Lösung mit $NH_3$ bei pH = 6 - 7 erhalten wird. Nach Abfiltrieren des Niederschlags wird bei 110 °C getrocknet und bei 500 °C calciniert. Katalysator S enthält 28,5 Gew.% Al und 13,2 Gew.% P.

Katalysator T

$SiO_2$ im Handel erhältlich unter D 11-10®.

Katalysator U

$TiO_2$ P 25® wird zu 2-mm-Strängen verformt, bei 110 °C getrocknet und bei 500 °C/16 h calciniert.

Katalysator V

D 10-10® wird mit $H_3BO_3$ imprägniert, getrocknet bei 110 °C und bei 500 °C/5 h calciniert. Der Katalysator V setzt sich zusammen aus 85 % $Al_2O_3$ und 15 % $B_2O_3$.

Katalysator W

Katalysator W erhält man, indem man D 10-10® $Al_2O_3$ mit 85 %iger $H_3PO_4$ 30 min behandelt, danach bei 30 °C/2 h trocknet und bei 540 °C/2 h calciniert. Der P-Gehalt beträgt 4,9 Gew.%.

Katalysator X

D 11-10® $SiO_2$ werden mit $H_3BO_3$ in methanolischer Lösung behandelt, bei 110 °C getrocknet und bei 500 °C/5 h calciniert. Der Borgehalt des Katalysators X beträgt 3,0 Gew.%.

Katalysator Y

200 g Katalysator T werden mit 600 ml 15 %iger HCl bei 80 °C/h behandelt. Danach wird das Material Cl-frei gewaschen, bei 110 °C getrocknet und bei 600 °C/1 h calciniert.

Katalysator Z

100 g des Katalysators T werden mit 200 ml einer 0,1 n HF bei 90 °C 2 h behandelt und nach Abfiltrieren F-frei gewaschen, bei 110 °C getrocknet und bei 600 °C/1 h calziniert.

Beispiele

Beispiel 1

Gasphasenpyrolyse von p-Methoxiisopropylbenzaldehyd zu p-Isopropenyl-benzaldehyd am Zeolith-Katalysator A

Die Pyrolyse erfolgt in einem beheizten Rohr aus Quarzglas, das mit 43 ml des Katalysators A in Strangform (1: ca. 8 mm, Ø: ca. 2 mm) beschickt war. Die im Inneren des Katalysatorbettes während der Reaktion gemessenen Temperaturen betragen 250 bis 350 °C, der Durchsatz an p-Methoxiisopropyl-benzaldehyd liegt bei 25 ml/h, die mittlere Verweilzeit, die durch Zudosieren von Inertgas eingeregelt werden kann, lag bei 1 bis 3 s. Umsatz an p-Methoxiisopropyl-benzaldehyd sowie Selektivität der Bildung von p-Isopropenyl-benzaldehyd werden durch gaschromatische Analyse (GC) ermittelt. Umsatz: quantitativ, Selektivität: 92,4 %, jeweils bei 250 °C.

Beispiele 2 bis 14

Gasphasenpyrolyse von p-Methoxiisopropyl-benzaldehyd zu p-Isopropenylbenzaldehyd an diversen Zeolith-Katalysatoren bzw. Phosphat-Katalysatoren mit Zeolithstruktur

Die Pyrolyse erfolgt in Analogie zu Beispiel 1. Experimentelle Bedingungen und Ergebnisse gehen aus Tabelle 1 hervor:

Tabelle 1

| Beispiele | 2 | 3 | 4 | 5 | 6 | 7 | 8 | 9 | 10 | 11 | 12 | 13 | 14 |
|---|---|---|---|---|---|---|---|---|---|---|---|---|---|
| Katalysator | B | C | D | E | F | G | H | I | J | K | L | M | N |
| Temperatur °C | 350 | 320 | 320 | 320 | 320 | 300 | 320 | 320 | 320 | 300 | 300 | 320 | 320 |
| Zulauf ml/h | 55 | 37 | 40 | 50 | 31 | 62 | 35 | 45 | 45 | 50 | 50 | 40 | 45 |
| Umsatz % | 88,6 | 99,2 | 94,2 | 99,7 | 99,3 | 96,5 | 97,8 | 96,3 | 74,5 | 74,1 | 96,5 | 80,8 | 49,0 |
| Selektivität | 70,7 | 86,6 | 93,3 | 70,8 | 95,6 | 96,5 | 95,5 | 82,7 | 67,0 | 74,7 | 97,5 | 79,6 | 85,7 |

Beispiel 15

Gasphasenpyrolyse von p-Methoxiisopropylbenzaldehyd-dimethylacetal am Zeolith-Katalysator A

Die Pyrolyse wird in Analogie zu Beispiel 1 im Temperaturbereich 250 bis 350 °C durchgeführt; zusätzlich speist man 6 bis 7 g/h Wasserdampf ein. Laut GC ist der Umsatz quantitativ, die Selektivität zu p-Isopropenylbenzaldehyd beträgt 92,7 %.

Beispiel 16

Gasphasenpyrolyse von p-Methoxiisopropyltoluol zu p-Isopropenyltoluol am Zeolith-Katalysator A

Die Pyrolyse erfolgt in einem beheizten Rohr aus $V_2A$-Stahl, das mit 370 ml Katalysator A in Strangform (1 = 2 bis 16 mm, Ø: ca. 2,8 mm) gefüllt ist. Die im Innern des Katalysatorbettes während der Reaktion gemessenen Temperaturen betragen 325 bis 404 °C, der Durchsatz an p-Methoxiisopropyltoluol liegt bei 200 g/h, entsprechend einer mittleren Verweilzeit von etwa 5 s.

Während der gesamten Versuchdauer von 516 h ist die Aktivität des Katalysators gleichbleibend hoch. Der Umsatz an p-Methoxiisopropyltoluol ist lt. GC ≥99,9 %, die Selektivität der Bildung von p-Isopropenyltoluol beträgt 98,0 bis 99,2 %.

Die mit den Katalysatoren P bis Z erzielten Versuchsergebnisse und Versuchsbedingungen sind in den folgenden Beispielen 17 bis 27 und in Tabelle 2 zusammengefaßt.

Beispiel 17

Gasphasenpyrolyse von p-Methoxiisopropylbenzaldehyd zu p-Isopropenyl-benzaldehyd an saurem Kieselgel (Katalysator T)

Die Pyrolyse erfolgt in einem beheizten Rohr aus Quarzglas, das mit 15 g des technischen Kieselgel-Kontaktes D 11-10 (BASF; Katalysator T in Strangform, 1: ca 8 mm Ø: ca. 2 mm) beschickt ist. Die im Inneren des Katalysatorbettes während der Reaktion gemessenen Temperaturen betragen 300 - 400 °C, der Durchsatz an p-Methoxiisopropyl-benzaldehyd liegt bei etwa 25 ml/h, die mittlere Verweilzeit, die durch Zudosieren von Inertgas eingeregelt werden kann, liegt bei 1 bis 3 s. Umsatz von p-Methoxiisopropylbenzaldehyd sowie Selektivität der Bildung von p-Isopropenyl-benzaldehyd werden durch gaschromatographische Analyse (GC) ermittelt: Umsatz: quantitativ, Selektivität: 94,6 %.

Beispiele 18 bis 27

Gasphasenpyrolyse von p-Methoxiisopropyl-benzaldehyd zu p-Isopropenyl-benzaldehyd an diversen sauren

Heterogenkatalysatoren

Die Pyrolysereaktionen erfolgen in Analogie zu Beispiel 17. Experimentelle Bedingungen und Ergebnisse gehen aus Tabelle 2 hervor.

Tabelle 2

| Beispiele | 18 | 19 | 20 | 21 | 22 | 23 | 24 | 25 | 26 | 27 |
|---|---|---|---|---|---|---|---|---|---|---|
| Katalysator | P | Q | R | S | T | V | W | X | Y | Z |
| Temperatur °C | 320 | 300 | 300 | 320 | 320 | 320 | 320 | 350 | 320 | 320 |
| Zulauf ml/h | 25 | 62 | 166 | 45 | 25 | 25 | 40 | 55 | 60 | 25 |
| Umsatz % | 44,3 | 90,0 | 95,7 | 91,5 | 93,2 | 96,4 | 54,0 | 92,0 | 60,3 | 70,2 |
| Selektivität | 85,0 | 88,7 | 98,7 | 83,7 | 89,4 | 86,3 | 85,3 | 68,1 | 90,6 | 80,4 |

Beispiel 28

Gasphasenpyrolyse von p-Methoxiisopropyl-benzaldehyddimethylacetal an Kieselgel D 11-10 (Katalysator T)

Die Pyrolyse wird in Analogie zu Beispiel 17 bei 270 °C und einem Durchsatz an p-Methoxiisopropylbenzaldehyddimethylacetal von etwa 35 ml/h durchgeführt.

Laut GC ist der Umsatz > 99,9 %; Selektivität der Bildung von p-Isopropenylbenzaldehyd: 47,5 %, von p-Isopropenylbenzyl-methylether: 23,7 % und von p-Isopropenylbenzoesäuremethylester: 13,7 %.

Beispiel 29

Gasphasenpyrolyse von p-Methoxiisopropylbenzaldehyd-dimethylacetal an Kieselgel D 11-10 (Katalysator T) unter Zudosierung von Wasserdampf

Die Pyrolyse wird in Analogie zu Beispiel 28 bei 300 °C durchgeführt; zusätzlich speist man 6 bis 7 g Wasserdampf/h in den Reaktoreingang ein. Laut GC ist der Umsatz quantitativ; im Vergleich zu Beispiel 28 verdoppelt sich die Selektivität der Bildung von p-Isopropenylbenzaldehyd nahezu auf 90,8 %.

Beispiel 30

Gasphasenpyrolyse von p-Methoxiisopropyltoluol zu p-Isopropenyltoluol an Kieselgel D 11-10 (Katalysator T)

Die Pyrolyse wird in Analogie zu Beispiel 17 bei 400 °C und einem Durchsatz an p-Methoxiisopropyltoluol von etw 25 ml/h durchgeführt.

Laut GC ist der Umsatz quantitativ, die Selektivität der Bildung von p-Isopropenyltoluol ist > 98 %.

**Ansprüche**

1. Verfahren zur Herstellung von Alkenylaromaten der allgemeinen Formel I

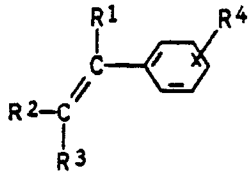

(I),

in der $R^1$ geradkettige oder verzweigte Alkylreste mit 1 bis 12 Kohlenstoffatomen oder Cycloalkylreste mit 5 bis 8 Kohlenstoffatomen oder Arylreste bedeutet, $R^2$ und $R^3$ gleich oder verschieden sind und Wasserstoff oder Reste wie unter $R^1$ genannt sein können, und in der $R^4$ für Wasserstoff, Kohlenwasserstoffreste wie $R^1$, Alkenylreste mit 2 bis 12 Kohlenstoffatomen, Alkoxireste mit 1 bis 12 Kohlenstoffatomen, Aryloxireste mit 6 bis 12 Kohlenstoffatomen, der Formylrest, Acylreste mit 2 bis 12 Kohlenstoffatomen, Acetalreste mit 3 bis 12 Kohlenstoffatomen, Esterreste mit 2 bis 12 Kohlenstoffatomen, der Cyanorest, Halogen oder einen Halogenalkylrest mit 1 bis 5 Kohlenstoffatomen, z.B. $-CF_3$, $-CCl_3$, $-CH_2CF_3$, steht, dadurch gekennzeichnet, daß man Alkoxialkylaromaten der allgemeinen Formel II

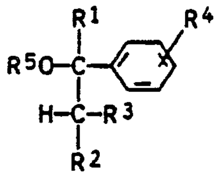

(II),

in der $R^1$ bis $R^4$ obige Bedeutung haben und $R^5$ für geradkettige oder verzweigte Alkylreste steht, in Gegenwart von aciden Heterogenkatalysatoren umsetzt.

2. Verfahren nach Anspruch 1, dadurch gekennzeichnet, daß man als acide Heterogenkatalysatoren saure Oxide, Phosphate, die gegebenenfalls Zeolithstruktur besitzen und/oder Zeolithe einsetzt.

3. Verfahren nach Anspruch 1, dadurch gekennzeichnet, daß man als Ausgangsstoff p-(2-Methoxi-2-propyl)-benzaldehyd, dessen Dimethylacetal oder p-(2-Methoxi-2-propyl)-toluol einsetzt.

4. Verfahren nach Anspruch 1, dadurch gekennzeichnet, daß man als Katalysatoren Zeolithe des Pentasiltyps verwendet.

5. Verfahren nach Anspruch 1, dadurch gekennzeichnet, daß man als Katalysatoren Aluminium-, Boro-, Gallium- und/oder Eisensilikatzeolithe des Pentasiltpys verwendet, die gegebenenfalls mit Alkalimetallen, Übergangsmetallen und/oder Seltenen Erdmetallen dotiert sind.

6. Verfahren nach Anspruch 1, dadurch gekennzeichnet, daß man als acide Heterogenkatalysatoren saure Metalloxide, Phosphate oder hydrothermal hergestellte Phosphate mit Zeolithstruktur verwendet.

7. Verfahren nach Anspruch 1, dadurch gekennzeichnet, daß man als acide Heterogenkatalysatoren saure Oxide der Elemente Si, Ti, Zr, B, Fe, W, Mo oder Phosphate der Elemente B, Al, Zr, Ce, Fe, Sr oder deren Gemische oder hydrothermal hergestellte Aluminium-, Silicium-aluminium-, Siliciumeisenaluminium- oder Boraluminiumphosphate mit Zeolithstruktur verwendet.

8. Verfahren nach Anspruch 1, dadurch gekennzeichnet, daß man als acide Heterogenkatalysatoren Phosphorsäure bzw. Borsäure auf den Trägermaterialien $SiO_2$, $Al_2O_3$, $TiO_2$ und Bims verwendet.

9. Verfahren nach Anspruch 1, dadurch gekennzeichnet, daß man als acide Heterogenkatalysatoren mit Übergangsmetallen oder Seltenen Erdmetallen dotierte Katalysatoren verwendet.

10. Verfahren nach Anspruch 1, dadurch gekennzeichnet, daß man die Reaktion unter Zusatz von Wasser oder Wasserdampf durchführt.